# EUROPEAN PATENT APPLICATION

(11) **EP 4 650 904 A1**
(43) Date of publication of application: **19.11.2025**
(21) Application number: 25176021.1
(22) Date of filing: 13.05.2025
(51) Int. Cl.: G05D 7/06, C25B 1/04, H02J 3/00, G05B 13/00, C01B 3/00

(54) **SYSTEM AND METHOD FOR STABILIZING THE OPERATION OF FACILITIES USING HYDROGEN PRODUCED BY LOW CARBON SOURCES**

(30) Priority: 14.05.2024 US 202463647500 P
(71) Applicant: Kellogg Brown & Root LLC, Houston, Texas 77002 (US)
(72) Inventor: PACHPANDE, Sunil Nivrutti, Houston, TX, 77002 (US); BANTWAL BALIGA, Satish, Houston, TX, 77002 (US); FENG, Zhentao, Houston, TX, 77002 (US); YAMALIDOU, Ekaterini, Houston, TX, 77002 (US)
(74) Representative: Weickmann & Weickmann PartmbB

(57) **Abstract**

A system and a method for stabilizing hydrogen flow to a downstream process in a facility determining a hydrogen density and pressure profiles in the hydrogen storage unit for different target net hydrogen flows at different time intervals of a time horizon of a renewable power availability profile, determining an operating target net hydrogen flow of a hydrogen feed to the downstream process, determining a target direct hydrogen flow of a hydrogen feed and a target stored hydrogen flow of a hydrogen feed to the downstream process, and controlling the operation of the downstream process based on the operating target hydrogen flows.

## Description

### CROSS-REFERENCE TO RELATED APPLICATIONS

This application claims the benefit of U.S. Provisional Patent Application No. 63/647,500, filed on May 14, 2024, the contents of which are incorporated herein by reference.

### TECHNICAL FIELD

The present disclosure relates to industrial processes using hydrogen produced by a low carbon energy source.

### BACKGROUND

Conventionally, hydrogen required by downstream processes such as ammonia production, methanol production, etc. is produced by processing a hydrocarbon feedstock via methods such as natural gas reforming, partial oxidation of hydrocarbons or methane pyrolysis, all of which generate carbon dioxide emissions. Alternative hydrogen sources are being adopted, such as an electrolyzer/electrolysis process, which only requires water and electricity. If a renewable energy source supplies the electricity for the electrolyzer, then hydrogen can be generated without carbon emissions. However, because the production processes using hydrogen require steady-state conditions such as invariant flowrate for the hydrogen feed, using renewable energy sources can be problematic when operating an electrochemical system such as an electrolyzer.

Renewable energy sources, such as wind or solar power, are prone to changes in environmental conditions; e.g., lulls in winds, inclement weather, etc. A reduction in wind speed or sunlight intensity can lead to reductions in available electrical power. Reduced electrical power, in turn, causes the hydrogen production source (e.g., electrolyzer) to generate less hydrogen feed, which can impair production of the final product.

Since the operating load of the downstream production process needs to be as stable as possible, the adjustments to the hydrogen flow to the downstream production process need to be minimized while simultaneously accounting for the current and future values of the renewable energy source based on the available renewable energy profile, other available sources of hydrogen and the available hydrogen inventory, which is required to operate within an allowable range of operating pressures.

### SUMMARY

Examples of a system and a method for stabilizing the operation of facilities using hydrogen produced by low carbon source as described may substantially obviate one or more of the problems due to limitations and disadvantages of the related art or at least to provide the public with a useful alternative.

In examples, described is a system and a method for stabilizing hydrogen flow to a downstream process in a facility comprising a hydrogen storage unit and powered by a low carbon energy source. In examples, described is a system and a method for stabilizing hydrogen flow to a downstream process in a facility determining a hydrogen density and pressure profiles in the hydrogen storage unit for different target net hydrogen flows at different time intervals of a time horizon of a renewable power availability profile, determining an operating target net hydrogen flow of a hydrogen feed to the downstream process, determining a target direct hydrogen flow of a hydrogen feed and a target stored hydrogen flow of a hydrogen feed to the downstream process, and controlling the operation of the downstream process based on the operating target hydrogen flows.

In examples, the system and method as described may enable efficient production while using a dynamic energy source, such as renewable energy, to provide energy to a process whose stability is affected by a dynamic energy supply.

In examples, determining a hydrogen density and pressure profiles in the hydrogen storage unit for different target net hydrogen flows at different time intervals of a time horizon of a renewable power availability profile may include determining a mass of the hydrogen produced at each time interval across the time horizon; determining a relationship between the pressure and the density of hydrogen in the hydrogen storage unit; determining a density of the hydrogen in the hydrogen storage unit at each target net hydrogen flow for each time interval for the time horizon; and determining the pressure profile of the hydrogen storage unit at each time interval of the time horizon.

In examples, the system and the method may include determining the hydrogen density and pressure profiles in the hydrogen storage unit for different target net hydrogen flows at different time intervals over a time horizon of a renewable power availability profile; determining a first target net hydrogen flow of a hydrogen feed to the downstream process for a given time interval within the time horizon, wherein the first target net hydrogen flow corresponds to the maximum time required for a pressure of the hydrogen in the hydrogen storage unit to breach the high-pressure or the low-pressure limit; determining a second target net hydrogen flow of a hydrogen feed to the downstream process for a given time interval within the time horizon, wherein the second target net hydrogen flow corresponds to a pressure profile of the hydrogen storage unit with the lowest deviation from the high-pressure or the low-pressure safety limits of the hydrogen storage unit; setting as operating target net hydrogen flow the greater of the first target net hydrogen flow and the second target net hydrogen flow; and controlling an operation of the downstream process based on the operating target net hydrogen flow.

In examples, controlling an operation of the downstream process based on the operating target net hydrogen flow may include sending the operating target net hydrogen flow to an advanced regulatory control system, a user interface or both.

In examples, the downstream process may include ammonia synthesis, methanol synthesis, or any other process that involves the use of renewable power and/or one or more hydrogen sources.

In examples, the system and the method may include a non-transitory computer readable medium having stored thereon computer-readable instructions that, when executed by a processor, cause the processor to: determine a hydrogen density and pressure profiles in the hydrogen storage unit for different target net hydrogen flows at different time intervals of a time horizon of a renewable power availability profile; determine a first target net hydrogen flow of a hydrogen feed to the downstream process for a given time interval within the time horizon, wherein the first target net hydrogen flow corresponds to a maximum time required for a pressure of the hydrogen in the hydrogen storage unit to breach high-pressure or low-pressure limit; determine a second target net hydrogen flow of a hydrogen feed to the downstream process for a given time interval within the time horizon, wherein the second target net hydrogen flow corresponds to a pressure profile of the hydrogen storage unit with the lowest deviation from a safety limit; set as operating target net hydrogen flow the greater of the first target net hydrogen flow and the second target net hydrogen flow; and apply the operating target net hydrogen flow to control operation of the downstream process.

In examples, the process to determine a hydrogen density and pressure profiles in the hydrogen storage unit for different target net hydrogen flows at different time intervals of a time horizon of a renewable power availability profile may include the processor to determine a mass of the hydrogen produced at each time interval across the time horizon; determine a relationship between the pressure and the density of hydrogen in the hydrogen storage unit; determine a density of the hydrogen in the hydrogen storage unit at each target net hydrogen flow for each time interval for the time horizon; and determine the pressure profile of the hydrogen storage unit at each time interval of the time horizon.

In examples, control of the operation of the downstream process may be based on the operating target net hydrogen flow comprises sending the operating target net hydrogen flow to an advanced regulatory control system, a user interface or both.

In examples, described is a system and a method for regulating the flow of hydrogen produced using a low carbon energy source to processes producing a product in a facility having a downstream production process unit. In examples, the downstream production process unit may include a unit for ammonia synthesis, methanol synthesis, or any other unit that involves the use of renewable power and/or one or more hydrogen sources. In examples, the system and the method may include supplying energy to the facility, wherein at least a portion of the supplied energy is from a low carbon energy source dependent upon at least one environmental parameter; estimating energy availability from the low carbon energy source over a selected time period using the at least one environmental parameter; forming a hydrogen feed to the downstream production process unit using at least one of: (i) a primary hydrogen feed generated by a hydrogen source energized by the low carbon energy source, and (ii) a supplemental hydrogen feed; controlling the forming of the hydrogen feed using an advanced regulatory controller (ARC), the ARC being configured to generate setpoints regarding the hydrogen feed using the estimated energy availability; and producing a product by feeding the formed hydrogen feed to the downstream production process unit, wherein the hydrogen feed to the downstream production process unit is stabilized by the process described herein.

In examples, described is a facility using a low carbon energy source, the facility may include a downstream production process unit; a low carbon energy source dependent upon at least one environmental parameter; a hydrogen source powered by the low carbon energy source; a hydrogen storage unit arranged to receive hydrogen from the hydrogen source; a hydrogen feed to the downstream production process unit and fluidly connected to the hydrogen storage unit; a system for stabilizing hydrogen flow to a downstream process as described herein; and a product output. In examples, the downstream production process unit is an ammonia synthesis unit, a methanol synthesis unit, or any other unit that involves the use of renewable power and/or one or more hydrogen sources..

It should be understood that certain features of the disclosure have been summarized rather broadly in order that the detailed description thereof that follows may be better understood, and in order that the contributions to the art may be appreciated. There are, of course, additional features of the disclosure that will be described hereinafter and which will in some cases form the subject of the claims appended thereto.

### BRIEF DESCRIPTION OF THE DRAWINGS

For detailed understanding of the present disclosure, references should be made to the following detailed description taken in conjunction with the accompanying drawings, in which like elements have been given like numerals and wherein:
FIG. 1 schematically illustrates a low carbon process for synthesizing end products by downstream processes using hydrogen produced by low carbon energy in accordance with one embodiment of the present disclosure.
FIG. 2 shows the pressure profile of hydrogen in the hydrogen storage unit 112 for iterative values of the target hydrogen flow for a power availability profile for a sample 24-hour horizon.
FIG. 3 shows the safety margin applied to the high and low operating pressure limits of the hydrogen storage unit 112.
FIG. 4 shows the description of an example of the method carried out by system 150.

### DETAILED DESCRIPTION

In aspects, the present disclosure provides systems and related methods for stabilizing and optimizing the hydrogen load to the downstream production units even when hydrogen is generated from a low carbon source (e.g. electrolyzer) with a high degree of variability due to variability to the available renewable energy profile.

There are shown in the drawings, and herein will be described in detail, specific embodiments of the present disclosure with the understanding that the present disclosure is to be considered an exemplification of the principles of the disclosure and is not intended to limit the disclosure to that illustrated and described herein.

In examples, the system and method as described herein may be implemented in a facility that includes a downstream process or downstream production process unit. The nature of the downstream process or downstream production process unit are not limiting. In examples, the downstream process may include ammonia synthesis, methanol synthesis, or other processes that involve the use of renewable power and/or one or more hydrogen sources. In examples, a downstream production process unit may include equipment for the production or synthesis of a material. In examples, the downstream production process unit includes one or more reactors. In examples, a downstream production process unit may include equipment in addition to one or more reactors, such as one or more heat exchangers or heaters, one or more separators, one or more flow pumps, or any combination thereof as may be desirable for the production or synthesis of the desired material. In examples, the downstream production process unit includes equipment configured for ammonia production or synthesis (e.g., an ammonia synthesis unit), equipment including a reactor configured for methanol production or synthesis (e.g., a methanol synthesis unit), or other production equipment that involves the use of renewable power and/or one or more hydrogen sources.

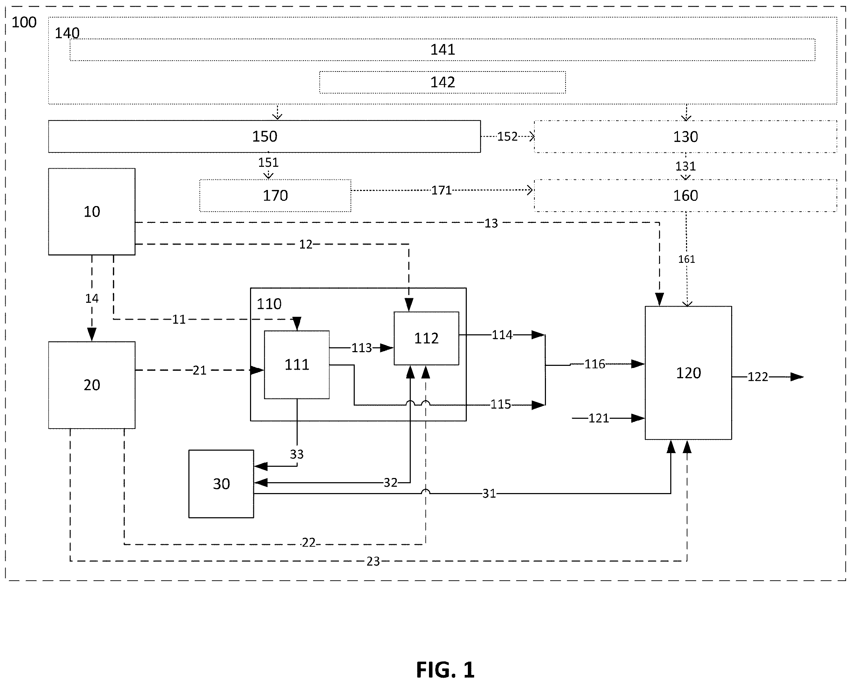

Referring to FIG. 1, there is schematically shown a non-limiting embodiment of a facility 100 in which the system and method as described may be implemented. In examples, facility 100 may include a downstream production process unit 120 that uses hydrogen. In examples, downstream production process unit 120 may also employ one or more feed streams 121. In examples, downstream production process unit 120 may produce one or more product streams 122. In examples, a product stream 122 may include ammonia or methanol. In examples, product stream 122 may include other materials.

In examples, to reduce or eliminate carbon emissions, a low carbon energy source 10 may be used to supply electrical energy to one or more components of facility 100. In examples, the power supply of the low carbon energy source 10 may be variable and/or inconsistent, thus affecting the power output of the low carbon energy source 10. Because the power output of the low carbon energy source 10 may fluctuate due to external influences, such as weather conditions, the supply of power from the low carbon energy source 10 may encounter prolonged interruptions or be subject to diminished capacity. In examples, the implementation of system and method as described herein, a low carbon energy source 10 may still be used to supply energy to facility 100 even if the low carbon energy source 10 does not always provide consistent and continuous power supply. Accordingly, it is emphasized that terms such as "supplying power" or "supplying energy" does not require an uninterrupted supply or power having any specified minimum requirements.

In examples, downstream production process unit 120 receives one or more feed streams 121. In examples, one or more feed streams 121 provide downstream production process unit 120 with one or more materials to react with hydrogen to produce a product stream 122. In examples, the downstream production process unit 120 receives a hydrogen feed 116. In examples, hydrogen feed 116 may be supplied by hydrogen supply line 115 fluidly connect to hydrogen feed 116. In examples, hydrogen supply line 115 may be directly supplied by a hydrogen source 111. The downstream production process unit 120 may be powered by the low carbon energy source 10 via the power supply line 12 and/or from a secondary energy source 20 (e.g., a power grid) via the power supply line 23. The secondary energy source 20 may also supply power to the hydrogen source 111 via the power supply line 21 and to the hydrogen storage unit 112, for example a storage tank, via the power supply line 22. The secondary energy source 20 may be operationally independent of facility 100. That is, the secondary energy source 20 is not controlled by or dependent on facility 100.

In examples, facility 100 may include a hydrogen plant 110. In examples, the hydrogen plant 110 may use a low carbon process to generate hydrogen for hydrogen source 111, from which the direct hydrogen supply line 115 is sourced. In examples, electricity for the hydrogen production process may be supplied by the low carbon energy source 10 via the power supply line 11. In one arrangement, the hydrogen plant 110 may include hydrogen source 111 and a hydrogen storage unit 112. In examples, the hydrogen source 111 may be an electrolyzer. The hydrogen source 111 may generate hydrogen for direct hydrogen supply line 115 that is fluidly connected to hydrogen feed 116 to the downstream production process unit 120 and/or for a storage hydrogen feed 113 to the hydrogen storage unit 112. The direct hydrogen supply line 115 and the storage hydrogen feed 113 are shown as separate merely for clarity. A common effluent line (not shown) from the hydrogen source 111 may be used to selectively direct flow of hydrogen to either or both of the downstream production process unit 120 and hydrogen storage unit 112. It should be noted that the present teachings are not limited to a hydrogen plant 110 that uses only an electrolyzer as the hydrogen source 111 to generate the hydrogen for direct hydrogen supply line 115 and/or storage hydrogen feed 113. The present teachings are equally applicable to any system or method of generating hydrogen via a low carbon process that uses electricity.

In examples, hydrogen storage unit 112 may provide a supplemental hydrogen feed for the downstream production process unit 120. In one arrangement, the hydrogen storage unit 112 stores hydrogen and supplies the stored hydrogen to the downstream production process unit 120, if needed, via a hydrogen supply line 114 that is fluidly connected to hydrogen feed 116. In examples, one or more valves (not shown) can be used to control the flow of hydrogen through the storage hydrogen feed 113 from the hydrogen source 111 to the hydrogen storage unit 112. Likewise, in examples, one or more valves (not shown) can be used to control the flow of hydrogen through the stored hydrogen supply line 114 from the hydrogen storage unit 112 to hydrogen feed 116 to the downstream production process unit 120.

The amount of hydrogen in the hydrogen storage unit 112 may vary depending on the amount of hydrogen produced by the hydrogen plant 110 and directed to the hydrogen storage unit 112 through the storage hydrogen feed 113 and on the amount of hydrogen directed to the downstream production process unit 120 via the stored hydrogen supply line 114.

In some embodiments, depending on the sources available to facility 100, the hydrogen storage unit 112 can be supplied by a secondary hydrogen source 30 via a secondary hydrogen feed 32. Also, excess hydrogen in hydrogen storage unit 112 can be exported to the secondary hydrogen source 30. The secondary hydrogen source 30 may also provide a supplemental hydrogen feed to the downstream production process unit 120. For example, hydrogen can be supplied to the downstream production process unit 120 directly from the secondary hydrogen source 30 via the direct secondary hydrogen feed 31. In examples, the secondary hydrogen source 30 may be a hydrogen source that is operationally independent of facility 100. That is, the secondary hydrogen source 30 may have access to sources of power and/or of hydrogen that are independent of facility 100.

In examples, to stabilize the operating load of the downstream production process unit 120, the adjustments to the direct hydrogen flow in hydrogen supply line 115 and the hydrogen supply line 114 from hydrogen storage unit 112 may be minimized while simultaneously accounting for the forecasted renewable energy profile and the amount of the hydrogen inventory in the hydrogen storage unit 112 that may be required to operate the downstream production process unit 120, even while the renewable power availability varies dynamically due to changes in weather, as well as day-night transitions.

In examples, facility 100 includes a system 150 as described herein. In examples, system 150 uses a multi-step iterative calculation to enable a stable, as well as a maximum hydrogen load to the downstream production process unit 120, when managing the variability of a renewable power profile and consequently with the variability in hydrogen production by the hydrogen plant 110. In examples, system 150 may include logic, computations, algorithms, schemas, microprocessors, memory modules, bi-directional signal transmission devices, display devices, input devices, and other components suitable for receiving, processing, storing, and transmitting information.

In examples, system 150 may include any number of logical, programmatic, and physical components. In examples, system 150 may include one or more processors and memory communicatively coupled with each other. In examples, one or more input/output devices such as monitors, keyboards, speakers, microphones, computer mouse and the like may be coupled to the one or more controllers. In examples, system 150 may include one or more communication elements such as receivers, transmitters, transceivers or like structure to enable wired and/or wireless communication.

In examples, memory associated with system 150 may be non-transitory computer-readable medium. Any suitable memory technology may be employed to implement a memory, for example, static random-access memory (SRAM), synchronous dynamic RAM (SDRAM), nonvolatile/Flash-type memory, or any other type of memory capable of storing information.

In examples, memory may be used to store logic instructions including, without limitation, one or more software modules and/or other sufficient information for operation, safety procedures, and/or routine maintenance processes. In examples, logic instructions may be employed to operate, control, and/or monitor the operation of the system and/or one or more subcomponents thereof. In examples, the memory may store an operating system and one or more software applications, instructions, programs, and/or data to implement the methods described herein and the functions attributed to the various systems. Any operation of the described system may be implemented in hardware, software, or a combination thereof. In the context of software, operations represent computer-executable instructions stored on one or more computer-readable storage media that, when executed by one or more processors, perform the recited operations. Computer-executable instructions may include programs, objects, routines, data structures, components, and the like that perform one or more functions or implement particular abstract data types.

In examples, system 150 receives or includes (e.g., stored in memory) information 140 relevant to the operation of facility 100. In examples, information 140 or any sub-portion thereof may be used in carrying out the determinations by system 150. In examples, the information 140 may include facility-specific information 141 and/or non-facility-specific information 142. Facility-specific information 141 may include operating parameters, such as current setpoints of the hydrogen source 111, hydrogen storage unit 112, downstream production process unit 120, other feeds required for the downstream production unit, and/or other component(s) associated with the facility 100. Facility-specific information 141 may also include operating parameters such as pressure, temperature, flow rates, energy usage, etc. Non-facility-specific information 142 may include environmental parameters, such as current weather conditions and weather forecasts, which may include information relating to temperature, wind speed, wind direction, gustiness, barometric pressure, precipitation, cloud cover, humidity, dew point, diurnal cycle, etc. This information may be current, historical and/or predicted. The non-facility-specific information may include non-weather-related information such as prevailing and expected energy usage in the immediate vicinity by other energy consumers, secondary energy source 20 availability, secondary hydrogen source 30 availability, etc. It should be noted that the facility-specific information and non-facility-specific information described above are only illustrative. The design and configuration of a facility 100, the geographical location in which the facility 100 is located, and the infrastructure in the vicinity of the facility 100 may require facility-specific information and/or non-facility-specific information that are not expressly listed above.

In examples, system 150 carries out iterative calculation to determine the current and future values of the pressure inside the hydrogen storage unit 112 across the time horizon for which the renewable power availability profile is given. In examples, using the current and future values of the pressure of the hydrogen storage unit 112 across the time horizon for which the renewable power variability profile, system 150 determines the target net hydrogen flow to the downstream production process unit 120 for the given renewable power availability profile and subsequently the target direct hydrogen flow in hydrogen supply line 115 and the target stored hydrogen flow in hydrogen supply line 114.

In examples, system 150 is configured to perform a rolling and dynamic calculation of the amount of hydrogen generated by the hydrogen source 111 at certain frequency across the duration of the renewable power availability profile. In examples, any frequency may be used that is not longer than the time step by which the power availability profile is renewed. This frequency should be long enough to maintain the operation of the downstream process and/or downstream production process unit as stable as possible. For purposes of this description, this frequency may be assumed to be equal to the frequency at which the renewable energy profile is updated. This is only an example.

The frequency of resetting the target net direct hydrogen flow for the hydrogen feed 116, and subsequently the target direct hydrogen flow in the hydrogen supply line 115 and the target stored hydrogen flow in hydrogen supply line 114, may depend on the volume of the hydrogen storage unit 112, the nameplate capacity of the downstream production process unit 120, and/or the time required for the pressure of the hydrogen storage unit 112 to reach its high or low constraint limit. In examples, system 150 may be configured to perform calculations to determine the magnitude and frequency of change in the maximum target net direct hydrogen flow for the hydrogen feed 116 to the downstream production process unit 120 and subsequently in the maximum target direct hydrogen flow in hydrogen supply line 115 and the maximum target stored hydrogen flow in hydrogen supply line 114, which may be based on thermodynamics and first principles.

In examples, system 150 is configured to predict the mass and pressure of the hydrogen in the hydrogen storage unit 112 over the time horizon for which the renewable power availability profile is given and may use this information to establish the maximum target net hydrogen flow for the hydrogen feed 116 to the downstream production process unit 120 and subsequently the target direct hydrogen flow in hydrogen supply line 115 and the target stored hydrogen flow in hydrogen supply line 114.

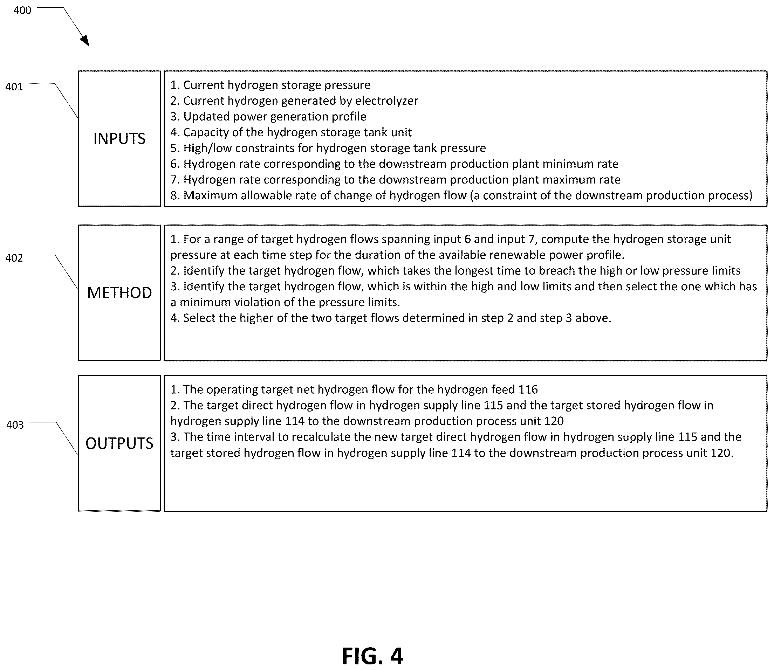

FIG. 4 illustrates a flowchart of an example of a process that may be carried out by system 150 as described in more detail below.

In examples, system 150 is configured to perform iterative calculations using different assumed values for the target net hydrogen flow for the hydrogen feed 116 to the downstream production process unit 120, spanning the range between the turndown capacity of the downstream production process unit 120 to its full name plate capacity. At each iterative calculation the target net direct hydrogen flow for the hydrogen feed 116 may be incrementally increased or decreased by a pre-determined amount, which may depend on the capacity of the downstream production process unit 120.

In examples, system 150 receives one or more of the following inputs 401 to use at each iteration of the calculation of the target net direct hydrogen flow for the hydrogen feed 116 to the downstream production process unit 120:
∘ the renewable power availability profile over a time horizon, which may be provided by the operator of the low carbon energy source 10,
∘ the pressure of the hydrogen storage unit 112 at the beginning of the same time horizon,
∘ the efficiency of the hydrogen source 111, defined as the rate of hydrogen production generated by the hydrogen source 111 as a function of available power,
∘ the capacity of hydrogen storage unit 112,
∘ the allowable range of operating pressures of the hydrogen storage unit 112,
∘ an assumed value for the target net hydrogen flow for the hydrogen feed 116 to the downstream production process unit 120.

As illustrated in FIG. 4, at 402, system 150 can then determine the target value for the net hydrogen flow for the hydrogen feed 116.

Step 1 - In examples, based on the efficiency of the hydrogen source 111 and an assumed value for the target net hydrogen flow for the hydrogen feed 116 to the downstream production process unit 120, system 150 determines the mass of the hydrogen produced at each time interval across the duration of the time horizon for which the renewable power generation profile is available. In this manner, system 150 can establish the profile of the hydrogen flow to the hydrogen storage unit 112 from the hydrogen source 111 as a function of time for the entire duration of the time period for which the renewable power profile is available.

Step 2 - In examples, system 150 determines the relationship between the pressure and the density of hydrogen in the hydrogen storage unit 112 based on gas law data at 40 deg C and a range of operating pressures between a low limit and a high limit of the hydrogen storage unit 112.

Step 3 - In examples, system 150 determines the density of the hydrogen in the hydrogen storage unit 112 at the beginning of the time horizon for which the renewable power availability profile is given based on the pressure of the hydrogen storage unit 112 at the beginning of the time horizon for which the renewable power availability profile is given. For each iterative calculation of the target net direct hydrogen flow for the hydrogen feed 116 to the downstream production process unit 120 system 150 may determine the density of hydrogen in hydrogen storage unit 112 at each time interval spanning the entire time horizon for which the renewable power availability profile is given. In examples, the density of the hydrogen in hydrogen storage unit 112 is determined based on the net change in hydrogen mass in the hydrogen storage unit 112 and the volume of the hydrogen storage unit 112. In this manner, at the beginning of the time horizon for which the renewable power availability profile is given system 150 can establish a hydrogen density profile in the hydrogen storage unit 112 for the entire time horizon for which the renewable power availability profile is given.

Step 4 - In examples, based on the hydrogen density profile in the hydrogen storage unit 112 as determined in step 3, system 150 can determine the pressure profile of the hydrogen storage unit 112 at each time interval for the entire duration of the time horizon for which the renewable power availability profile is given. In examples, the pressure profile of the hydrogen storage unit 112 may be determined based on regressing gas law data obtained from first principles as described in step 2 above.

In examples, via steps 3 and 4 above system 150 establishes the hydrogen density and pressure profiles in the hydrogen storage unit 112 at each time interval for the entire duration of the time horizon for which the renewable power availability profile is given for each assumed value of the target net hydrogen flow for the hydrogen feed 116 to the downstream production process unit 120 at each iteration.

In examples, system 150 may repeat steps 1 through 4 above for each successive assumed value for the target net hydrogen flow for the hydrogen feed 116 to the downstream production process unit 120. In this manner, system 150 can establish the hydrogen density and pressure profiles in the hydrogen storage unit 112 for the entire duration of the time horizon for which the renewable power availability profile is given.

In examples, based on the derived density and pressure profiles of hydrogen storage unit 112 for the entire duration of the time horizon for which the renewable power availability profile is given, system 150 can determine the value of the targe net hydrogen flow of the hydrogen feed 116 for downstream production process unit 120 for a given time interval.

In examples, in making this determination, system 150 may consider a safety margin of the hydrogen storage unit 112. In examples, the safety margins of the hydrogen storage unit 112 may be defined by a user and/or be previously uploaded to system 150. In examples, system 150 may apply a safety margin to the high and low operating pressure limits of the hydrogen storage unit 112.

In examples, to determine the value of the target net hydrogen flow of the hydrogen feed 116 for downstream production process unit 120 for a given time interval, system 150 considers at least one of the two criteria described below, for selecting the best target hydrogen flow to the downstream production unit.

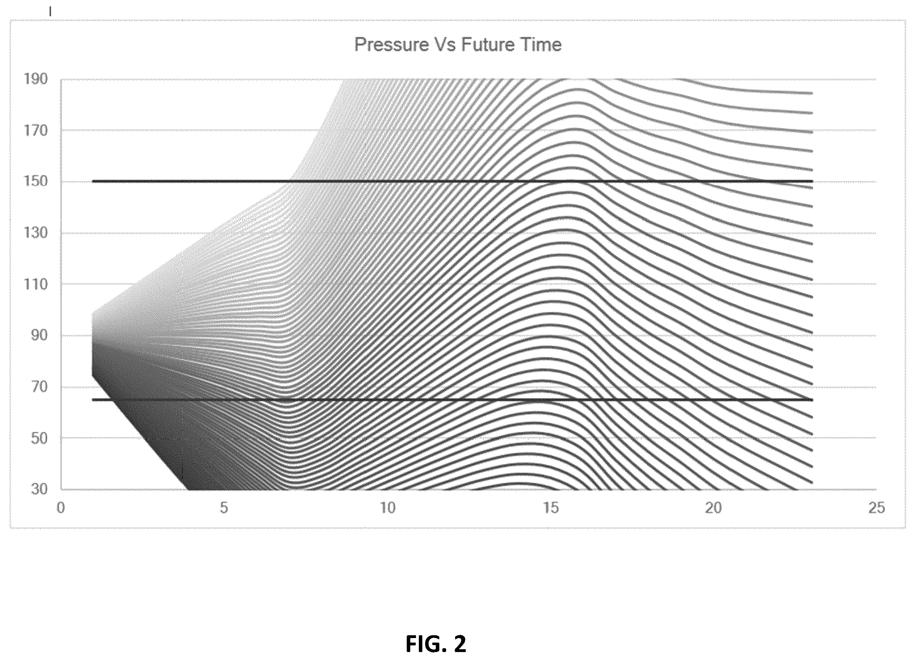

Criterion 1 - For each iterative target hydrogen flow calculation, system 150 analyzes the pressure profile over the time horizon of the renewable energy profile and plot the pressure profile across the time horizon, as shown in FIG. 2. In examples, system 150 compares each calculated value of the pressure and compare it to the pressure value of the design high pressure or low-pressure limits of the hydrogen storage unit 112. In examples, system 150 determines the time required for the pressure of the hydrogen in the hydrogen storage unit 112 to breach the high-pressure or low-pressure limit. In examples, these determined time values may be stored in an array. In examples, system 150 then selects the target net hydrogen flow for the hydrogen feed 116 to the downstream production process unit 120 as the maximum value of all elements of the array.

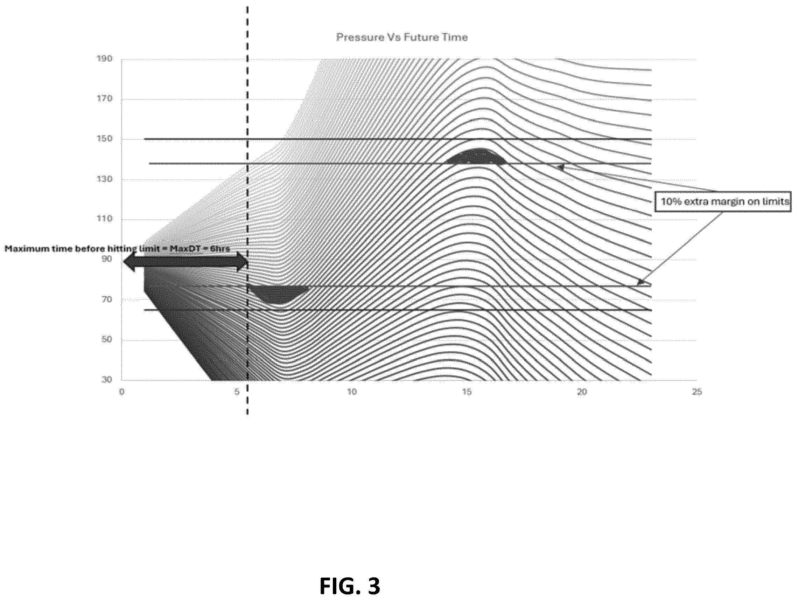

Criterion 2 - For each iterative calculation using assumed values of target hydrogen flow, system 150 determines the total deviation (area under the curve, shown as shaded area in FIG. 3) of the pressure profile of the hydrogen storage unit 112 over the time horizon of the renewable power profile from the allowable pressure limits calculated based on a user-defined safety margin (plus or minus) from the design high and low safety pressure limits of the hydrogen storage unit 112. In examples, system 150 stores the calculated values of the total deviation (area under the curve) of each pressure profile from the safety high or low limits in an array. The result of each of these calculations represents each instance in time when the pressure profile of the hydrogen storage unit 112 intersects high or low safety pressure limits of the hydrogen storage unit 112. In examples, system 150 determines as the best iterative target net hydrogen flow for the hydrogen feed 116 the one for which the corresponding pressure profile has the lowest deviation (i.e. smallest area under the curve) from the safety limits.

In examples, system 150 selects as the operating target net hydrogen flow to the downstream production process unit 120 the greater of the two target hydrogen flows which may have been calculated by criterion 1 and criterion 2.

In examples, system 150 determines the target flow values for the stored hydrogen flow in hydrogen supply line 114 based on keeping the hydrogen pressure in the hydrogen storage unit 112 within a range of pressures that may result in the most economic operation of the hydrogen storage unit 112. The value for this range may be an input to system 150, based on design parameters. In examples, based on the determined target stored hydrogen flow in hydrogen supply line 114 and the target net hydrogen flow for the hydrogen feed 116 to the downstream production process unit 120, system 150 determines the target flow value for the direct hydrogen supply line 115 as the difference between the target net hydrogen flow for the hydrogen feed 116 and stored hydrogen flow in hydrogen supply line 114.

In examples, system 150 is configured to automatically recalculate the target net hydrogen flow for the hydrogen feed 116 to the downstream production process unit 120 to account for an updated renewable power generation profile.

In examples, a plant operator using user interface 170 may override the automatic recalculation of the target net hydrogen feed 116 and may trigger manually system 150 to recalculate the target direct hydrogen flow in hydrogen supply line 115 and the target stored hydrogen flow in hydrogen supply line 114 to the downstream production process unit 120 at any time, if required for plant operation.

In examples, at 403, system 150 produces one or more of the following outputs:
1. The operating target net hydrogen flow for the hydrogen feed 116
2. The target direct hydrogen flow in hydrogen supply line 115 and the target stored hydrogen flow in hydrogen supply line 114 to the downstream production process unit 120
3. The time interval to recalculate the new target direct hydrogen flow in hydrogen supply line 115 and the target stored hydrogen flow in hydrogen supply line 114 to the downstream production process unit 120.

In examples, the outputs of system 150 are used to generate setpoints for the operation of facility 100.

In examples, facility 100 may include an advanced regulatory control system (hereafter ARC) 130. In examples, the ARC 130 may include logic, computations, algorithms, schemas, microprocessors, memory modules, bi-directional signal transmission devices, display devices, input devices, and other components suitable for receiving, processing, storing, and transmitting information.

In examples, ARC 130 may be configured to determine one or more setpoints 131 for controlling one or more operations of facility 100. The ARC 130 may determine the setpoint(s) based on the forecasted renewable energy profile for the low carbon energy source 10 and/or the energy availability from the secondary energy source 20. The ARC 130 may also determine the setpoint(s) 131 based on the target net hydrogen flow for the hydrogen feed 116 to the downstream production process unit 120. As used herein, a 'setpoint' is a value associated with a desired output, response, behavior, or operating state of a process component. A setpoint may be a value, range of values, an upper limit or a lower limit. By 'control,' it is meant modulating, stopping, starting, adjusting, increasing, decreasing, and/or maintaining one or more states, conditions, and/or parameters relating to a given operation. As further described below, the setpoint(s) 131 are transmitted to the distributed control systems (hereafter 'DCS') 160 of the facility 100.

In examples, the ARC 130 may determine one or more setpoints 131 using information 140.

In examples, the ARC 130 may determine and/or control increasing or decreasing the flow of hydrogen from the primary hydrogen source 111 to the hydrogen storage unit 112, increasing or decreasing the flow of hydrogen from the primary hydrogen source 111 to the downstream production process unit 120, and/or increasing or decreasing the flow of hydrogen from the hydrogen storage unit 112 to the downstream production process unit 120.

In examples, the ARC 130 may establish one or more setpoints 131 for facility 100 aiding in maintaining the downstream process and/or downstream production process unit stable even when the forecasted energy profile for the low carbon energy source 10 shows high degree of variability over a period of time. When establishing one or more setpoints 131, the ARC 130 may take into account one or more of facility-specific information 141 such as the minimum power requirements for the operation of all equipment of facility 100, the operating limits of all equipment of facility 100, including the allowable rate of change and the minimum turndown ratio of the downstream production process unit 120, and the availability of hydrogen in the hydrogen storage unit 112. When establishing one or more setpoints 131, the ARC 130 may also consider one or more of non-facility-specific information 142, such as the availability of power from the secondary energy source 20, the availability of hydrogen from the direct secondary hydrogen feed 115 and the requirements for the downstream production process unit 120.

The ARC 130 may also be able to address any dynamic mismatches between the forecasted power profile and the actual available renewable energy amount that may cause violations in the hydrogen storage unit pressure or in the operations of equipment within acceptable limits in the downstream.

In examples, system 150 is utilized in conjunction with an appropriate advanced process control strategy. In examples, ARC 130 receives one or more outputs of system 150 to establish the one or more setpoints 131. For example, the ARC 130 may receive via 152 an operating target for the hydrogen flow for the hydrogen feed 116 determined by system 150. In examples, the ARC 130 can generate one or more setpoints 131 to pass to the distributed control system (hereafter DCS) 160 for the control of the downstream production process unit 120, based on the target for hydrogen flow for the hydrogen feed 116 it receives from system 150. In examples, the ARC 130 can predict whether the operating target for the hydrogen flow determined by system 150 violates any downstream process operational constraints. If the operational constraints are not violated, ARC 130 can adopt the operating target value communicated at 152 as a setpoint and pass it to the DCS 160 as 131. In cases where the ARC 130 determines that the target for the hydrogen flow determined by system 150 violates a downstream process operational constraint, the ARC 130 can adjust the operating target for the hydrogen flow by changing the setpoint of a controller of the downstream production process unit 120 and may generate and pass the appropriate revised target setpoints as 131 to the DCS 160 of the downstream production process unit 120. In turn, DCS 160 may send a control signal 161 to the downstream production process unit 120.

In examples, the outputs of system 150 are passed to a plant operator via 151 to a user interface 170. In examples, the outputs of system 150 may be used as operating targets for the green hydrogen flow for the hydrogen feed 116 to the downstream production process unit 120. In examples, using user interface 170, an operator may manually communicate setpoints and/or setpoint adjustments as 171 to the DCS 160 regarding the amount of hydrogen flow to the downstream production process unit 120. In turn, DCS 160 may send control signals 161 to the downstream production process unit 120.

In examples, system 150 is configured to further stabilize the operating load of the downstream production process unit 120, by making the minimal adjustments to the hydrogen flow, while simultaneously accounting for the current and future values of the renewable energy source (based on the weather forecast) and the hydrogen inventory in the storage drum that is required to operate within an allowable range of operating pressures.

In examples, system 150 predicts the magnitude of the change required to the target net hydrogen flow for the hydrogen feed 116 that will help stabilize the operations of the downstream production process unit 120 even while the renewable power source varies dynamically due to changes in weather as well as day-night transitions.

In examples, a secondary energy source 20 may be available to facility 100 in some situations. The secondary energy source 20 may include an electrical grid. It should be noted that any surplus electricity generated by the low carbon energy source 10 may be fed into the electrical grid through energy connection 14. The secondary energy source 20 may also include a battery bank that is charged by the low carbon energy source 10 and/or the electrical grid through energy connection 24. As noted above, a secondary hydrogen source 30 may be available to facility 100 in some situations. When present, the secondary hydrogen source 30 may provide a supplement hydrogen feed to the downstream production process unit 120. This supplemental hydrogen feed may be in place of or in addition to the supplemental hydrogen supply line 114 from the hydrogen storage unit 112. It should be noted that any surplus hydrogen generated by the hydrogen source 111, or other hydrogen producing equipment, may be fed into the secondary hydrogen source 30 through the secondary hydrogen feed 33 or some other fluid line. The ARC 130 may regulate the use of energy from the secondary energy source 20 and the use of hydrogen from the secondary hydrogen source 30, depending on the low carbon power availability profile and the production of hydrogen by the hydrogen source 111.

As discussed previously, the non-facility-specific information 142 may relate to one or more environmental parameters. In examples, system 150 also receives facility-specific information 141. The facility-specific information 141 may relate to operating parameters discussed previously.

It should be noted that the equipment, devices, components, and systems described above are only exemplary of the equipment, devices, components, and systems designed and configured to perform their respective tasks. For example, an electrolyzer is only one example of a device that may be used to generate hydrogen. Other hydrogen sources may utilize renewable liquid reforming, high-temperature water-splitting, photobiological water splitting, photoelectrochemical water splitting, etc. Thus, the present teachings are neither limited to the equipment, devices, components, and systems described above nor the processes used therein.

As used herein, the term "low carbon energy" refers to an energy source that does not use hydrocarbons as the principal source of energy. Examples of low carbon power sources include, but are not limited to, solar power, wind power, tidal power, geothermal power, hydroelectric power, nuclear power, and hydrogen. It should be noted that the term "low carbon energy" source encompasses power sources that do not emit any carbon, i.e., "a zero carbon energy source."

The words "comprising" and "comprises" as used throughout the claims, are to be interpreted to mean "including but not limited to" and "includes but not limited to", respectively.

To the extent used herein, the word "substantially" shall mean "being largely but not wholly that which is specified."

As used herein, the singular forms "a," "an," and "the" are intended to include the plural forms as well, unless the context clearly indicates otherwise.

To the extent used herein, the term "about" in reference to a given parameter is inclusive of the stated value and has the meaning dictated by the context (e.g., it includes the degree of error associated with measurement of the given parameter).

To the extent used herein, the term "and/or" includes any and all combinations of one or more of the associated listed items.
The foregoing description is directed to particular embodiments of the present disclosure for the purpose of illustration and explanation. It will be apparent, however, to one skilled in the art that many modifications and changes to the embodiment set forth above are possible without departing from the scope of the disclosure. It is intended that the following claims be interpreted to embrace all such modifications and changes.

## Claims

1. A method for stabilizing hydrogen flow to a downstream process in a facility comprising a hydrogen storage unit and powered by a low carbon energy source comprising:
determining a hydrogen density and pressure profiles in the hydrogen storage unit for different target net hydrogen flows at different time intervals over a time horizon of a renewable power availability profile;
determining a first target net hydrogen flow of a hydrogen feed to the downstream process for a given time interval within the time horizon, wherein the first target net hydrogen flow corresponds to a maximum time required for a pressure of the hydrogen in the hydrogen storage unit to breach high-pressure or low-pressure limit;
determining a second target net hydrogen flow of the hydrogen feed to the downstream process for a given time interval within the time horizon, wherein the second target net hydrogen flow corresponds to a pressure profile of the hydrogen storage unit with the lowest deviation from a safety limit;
setting as operating target net hydrogen flow the greater of the first target net hydrogen flow and the second target net hydrogen flow; and
controlling an operation of the downstream process based on the operating target net hydrogen flow.

2. The method of claim 1, wherein determining a hydrogen density and pressure profiles in the hydrogen storage unit for different target net hydrogen flows at different time intervals of a time horizon of a renewable power availability profile comprises:
determining a mass of the hydrogen produced at each time interval across the time horizon;
determining a relationship between the pressure and the density of hydrogen in the hydrogen storage unit;
determining a density of the hydrogen in the hydrogen storage unit at each target net hydrogen flow for each time interval for the time horizon; and
determining the pressure profile of the hydrogen storage unit at each time interval of the time horizon.

3. The method of claim 1, controlling an operation of the downstream process based on the operating target net hydrogen flow comprises sending the operating target net hydrogen flow to an advanced regulatory control system, a user interface or both.

4. The method of claim 1, wherein the downstream process comprises ammonia synthesis or methanol synthesis.

5. A system for stabilizing hydrogen flow to a downstream process in a facility comprising a hydrogen storage unit and powered by a low carbon energy source, the system comprising:
a non-transitory computer readable medium having stored thereon computer-readable instructions that, when executed by a processor, cause the processor to:
determine a hydrogen density and pressure profiles in the hydrogen storage unit for different target net hydrogen flows at different time intervals of a time horizon of a renewable power availability profile;
determine a first target net hydrogen flow of a hydrogen feed to the downstream process for a given time interval within the time horizon, wherein the first target net hydrogen flow corresponds to a maximum time required for a pressure of the hydrogen in the hydrogen storage unit to breach high-pressure or low-pressure limit;
determine a second target net hydrogen flow of a hydrogen feed to the downstream process for a given time interval within the time horizon, wherein the second target net hydrogen flow corresponds to a pressure profile of the hydrogen storage unit with the lowest deviation from a safety limit;
set as operating target net hydrogen flow the greater of the first target net hydrogen flow and the second target net hydrogen flow; and
apply the operating target net hydrogen flow to control operation of the downstream process.

6. The system of claim 5, wherein determine a hydrogen density and pressure profiles in the hydrogen storage unit for different target net hydrogen flows at different time intervals of a time horizon of a renewable power availability profile comprises:
determine a mass of the hydrogen produced at each time interval across the time horizon;
determine a relationship between the pressure and the density of hydrogen in the hydrogen storage unit;
determine a density of the hydrogen in the hydrogen storage unit at each target net hydrogen flow for each time interval for the time horizon; and
determine the pressure profile of the hydrogen storage unit at each time interval of the time horizon.

7. The system of claim 5, wherein control operation of the downstream process based on the operating target net hydrogen flow comprises sending the operating target net hydrogen flow to an advanced regulatory control system, a user interface or both.

8. The system of claim 5, wherein the downstream process comprises ammonia synthesis or methanol synthesis.

9. A method for producing a product using a low carbon energy source, the product being produced by a facility having a downstream production process unit, the method comprising:
supplying energy to the facility, wherein at least a portion of the supplied energy is from a low carbon energy source dependent upon at least one environmental parameter;
estimating energy availability from the low carbon energy source over a selected time period using the at least one environmental parameter;
forming a hydrogen feed to the downstream production process unit using at least one of:
(i) a primary hydrogen feed generated by a hydrogen source energized by the low carbon energy source, and
(ii) a supplemental hydrogen feed;
controlling the forming of the hydrogen feed using an advanced regulatory controller (ARC), the ARC being configured to generate setpoints regarding the hydrogen feed using the estimated energy availability; and
producing a product by feeding the formed hydrogen feed to the downstream production process unit,
wherein the hydrogen feed to the downstream production process unit is stabilized by the process of claim 1.

10. The method of claim 9, wherein the product is ammonia or methanol.

11. A facility using a low carbon energy source, the facility comprising:
a downstream production process unit;
a low carbon energy source dependent upon at least one environmental parameter;
a hydrogen source powered by the low carbon energy source;
a hydrogen storage unit arranged to receive hydrogen from the hydrogen source;
a hydrogen feed to the downstream production process unit and fluidly connected to the hydrogen storage unit;
a system for stabilizing hydrogen flow to a downstream process; and
a product output,
wherein the system for stabilizing hydrogen flow is configured to:
determine a hydrogen density and pressure profiles in the hydrogen storage unit for different target net hydrogen flows at different time intervals of a time horizon of a renewable power availability profile;
determine a first target net hydrogen flow of a hydrogen feed to the downstream process for a given time interval within the time horizon, wherein the first target net hydrogen flow corresponds to a maximum time required for a pressure of the hydrogen in the hydrogen storage unit to breach high-pressure or low-pressure limit;
determine a second target net hydrogen flow of a hydrogen feed to the downstream process for a given time interval within the time horizon, wherein the second target net hydrogen flow corresponds to a pressure profile of the hydrogen storage unit with the lowest deviation from a safety limit;
set as operating target net hydrogen flow the greater of the first target net hydrogen flow and the second target net hydrogen flow; and
apply the operating target net hydrogen flow to control operation of the downstream process.

12. The facility of claim 11, wherein the downstream production process unit comprises an ammonia synthesis unit or a methanol synthesis unit.
